# EUROPEAN PATENT SPECIFICATION

(11) **EP 2 407 545 B1**
(45) Date of publication and mention of the grant of the patent: **06.07.2016**
(21) Application number: 10750256.9
(22) Date of filing: 10.03.2010
(51) Int. Cl.: C12N 15/76, A61P 33/10, A61P 31/06, A61P 35/00

(54) **AUXOTROPHIC, RECOMBINANT BCG STRAIN PASTEUR AND USE THEREOF FOR COMBATING HUMAN INFECTIONS CAUSED BY PARASITES**
AUXOTROPHER REKOMBINANTER BCG-STAMM PASTEUR UND SEINE VERWENDUNG ZUR BEKÄMPFUNG VON PARASITENBEDINGTEN INFEKTIONEN BEIM MENSCHEN
SOUCHE DE BCG PASTEUR AUXOTROPHE RECOMBINANT ET SON UTILISATION POUR LUTTER CONTRE DES INFECTIONS HUMAINES CAUSÉES PAR DES PARASITES

(30) Priority: 11.03.2009 BR PI0900896
(43) Date of publication of application: 18.01.2012
(73) Proprietor: Fundação Oswaldo Cruz, 21040-900, Rio de Janeiro, RJ (BR)
(72) Inventor: TENDLER, Miriam, Cep: 21040-900 Rio de Janeiro, RJ (BR); VILAR, Mônica Magno, Cep: 21040-900 Rio de Janeiro, RJ (BR); ARMÔA, Geraldo Rodrigues Garcia, Cep: 21040-900 Rio de Janeiro, RJ (BR); MCINTOSH, Douglas, Cep: 21040-900 Rio de Janeiro, RJ (BR); SIMPSON, Andrew, J., G., Cep: 21040-900 Rio de Janeiro, RJ (BR)
(74) Representative: Fritz, Edmund Lothar
(86) International application number: PCT/BR2010/000065
(87) International publication number: WO 2010/102363

(56) References cited:
- WO-A2-2004/067698
- DIETRICH G ET AL: "Novel vaccination strategies based on recombinant Mycobacterium bovis BCG", INTERNATIONAL JOURNAL OF MEDICAL MICROBIOLOGY, URBAN UND FISCHER, DE, vol. 292, no. 7-8, 1 January 2003 (2003-01-01), pages 441-451, XP004959935, ISSN: 1438-4221, DOI: 10.1078/1438-4221-00227
- BASTOS R G ET AL: "Recombinant Mycobacterium bovis BCG", VACCINE, ELSEVIER LTD, GB, vol. 27, no. 47, 5 November 2009 (2009-11-05), pages 6495-6503, XP026722047, ISSN: 0264-410X, DOI: 10.1016/J.VACCINE.2009.08.044 [retrieved on 2009-08-29]
- BORSUK, S. ET AL.: 'Auxotrophic complementation as a selectable marker for stable expression of foreign antigens in Mycobacterium bovis BCG.' TUBERCULOSIS vol. 87, no. 6, November 2007, pages 474 - 480, XP022301529
- VARALDO, P.B. ET AL.: 'Recombinant Mycobacterium bovis BCG expressing the Sm 14 antigen of Schistosoma mansoni protects mice from cercarial challenge.' INFECTION AND IMMUNITY vol. 72, no. 6, June 2004, pages 3336 - 3343, XP055060353
- ALMEIDA, M.S. ET AL.: 'Vaccination against Fasciola hepatica infection using a Schistosoma mansoni defined recombinant antigen, Sm14.' PARASITE IMMUNOLOGY vol. 25, March 2003, pages 135 - 137, XP055060653
- TENDLER M. ET AL.: 'A Schistosoma mansoni fatty acid-binding protein. Sml4, is the potential basis of a dual-purpose anti-helminth vaccine.' PROC. NATL. ACAD. SCI USA vol. 93, 1996, pages 269 - 273, XP002641579
- VILAR M.M. ET AL.: 'An experimental bivalent peptide vaccine against schistosomiasis and fascioliasis.' VACCINE vol. 22, 2003, pages 137 - 144, XP004471129

### SEQUENCE LISTING

<110> Fundação Oswaldo Cruz
<120> AUXOTROPHIC RECOMBINANT BCG STRAIN PASTEUR AND USE THEREOF FOR COMBATING HUMAN INFECTIONS CAUSED BY PARASITES
<130> P1733
<150> PCT/BR2010/000065
   <151> 2009-03-10
<160> 4
<170> PatentIn version 3.5
<210> 1
   <211> 22
   <212> DNA
   <213> Mycobacterium bovis
<400> 1
   aatctagaac agctagggga tc 22
<210> 2
   <211> 20
   <212> DNA
   <213> Mycobacterium bovis
<400> 2
   tcctgcagtt ctacgcctca 20
<210> 3
   <211> 35
   <212> DNA
   <213> Schistosoma mansoni
<400> 3
   tatatatata tataggcgcc accacaacga cgcgc 35
<210> 4
   <211> 28
   <212> DNA
   <213> Schistosoma mansoni
<400> 4
   cgcgggcgcc ttaggatgat cgtttata 28

## Description

### Field of the Invention

The present invention refers to a recombinant vaccinal strain of Mycobacterium bovis BCG expressing the Sm14 antigen of Schistosoma mansoni. The vaccinal strain of the present invention is employed to control infections caused by parasites, specifically Schistosoma mansoni. The vaccinal strain is an auxotrophic strain complemented to leucine derived from the genetic modification of BCG Pasteur sub-strain.

### Background of the Invention

It is estimated that at least 200 million people are infected with one of the five Schistosoma species affecting man. Besides, there are another 600 million at risk of being infected by this parasite.

Chemotherapy with the anti-helminthic drug Praziquantel is the main control method, however, the rate of re-infection is high [Al-Sherbiny M, Osman A, Barakat R, EI Morshedy H, Bergquist R & Olds R (2003). In vitro cellular and humoral responses to Schistosoma mansoni vaccine candidate antigens. Acta Trop. 88: 117-130 ; and Capron A, Riveau GJ, Bartley PB & McManus DP (2002). Prospects for a schistosome vaccine. Curr Drug Targets Immune Endocr Metabol Disord. 2: 281-290].

Therefore, the development of an effective vaccine used in combination with appropriate chemotherapy could represent an effective long-term control strategy [Pearce EJ (2003) Progress towards a vaccine for schistosomiasis. Acta Trop. 86: 309-313 ; and Bergquist NR (2002) Schistosomiasis: from risk assessment to control. Trends Parasitol. 18: 309-314 .].

The World Health Organization (WHO) selected the Schistosoma mansoni fatty acid-binding protein 14 (Sm14) and the 28-kDa glutathione S-transferase from S. haematobium (Sh28-GST), as priority target molecules for human clinical trials as anti-schistosome vaccines candidates [Bergquist, Feb 2004, http://who.int/tdr/publication/tdrnews/news71/schisto.html); Bergquist R, Al-Sherbiny M, Barakat R & Olds R. (2002) Blueprint for schistosomiasis vaccine development. Acta Trop. 82: 183-192 ; and, Bergquist NR (1998) Schistosomiasis vaccine development: progress and prospects. Mem Inst Oswaldo Cruz. 93 Suppl 1: 95-101.]. The selection of the Sm14 antigen was based on the observation that recombinant Sm14 protein, produced in Escherichia coli, induced protective immunity against S. mansoni (35 to 60%) and Fasciola hepatica, as determined by reduction in worm burden upon challenge in rodent models (Vilar, M. M.; Barrientos, F; Almeida, M; Garrat, R; Tendler, M. An Experimental Bivalent Peptide Vaccine against schistosomiasis and Fascioliasis with r-Sm14 peptides. Vaccine, v. 22, p. 137-144, 2003 ; and Tendler M, Brito CA, Vilar MM, Serra-Freire N, Diogo CM, Almeida MS, et al. (1996) A Schistosoma mansoni fatty acid-binding protein, Sm14, is the potential basis of a dual purpose anti-helminth vaccine. Proc Natl Acad Sci USA. 93: 269-273 .] and in sheep (Almeida MS, Torloni H, Lee-Ho P, Vilar MM, Thaumaturgo N, Simpson AJ & Tendler M (2003) Vaccination against Fasciola hepatica infection using a Schistosoma mansoni defined recombinant antigen, Sm14. Parasite Immunol. 25: 135-137]. In addition, the protective immunogenicity of Sm14 has been confirmed when the encoding gene of this protein was administered in the context of a DNA vaccine [Nascimento E, Lea-o IC, Pereira VR, Gomes YM, Chikhlikar P, August T, et al (2002) Protective immunity of single and multi-antigen DNA vaccines against schistosomiasis. Mem Inst Oswaldo Cruz. 97 Suppl 1: 105-109] or via live-attenuated recombinant bacteria i.e. Salmonella enterica var. Typhimurium (Pacheco LG, Zucconi E, Mati VL, Garcia RM, Miyoshi A, Oliveira SC, et al., (2005) Oral administration of a live Aro attenuated Salmonella vaccine strain expressing 14-kDa Schistosoma mansoni fatty acid-binding protein induced partial protection against experimental schistosomiasis. Acta Trop. 95: 132-142 .] or as the BCG recombinant obtained from the transformation of BCG strain of Mycobacterium bovis (Bacille Calmette-Guérin) used worldwide a vaccine against tuberculosis (Varaldo PB, Leite LC, Dias WO, Miyaji EN, Torres FI, Gebara VC, et al (2004) Recombinant Mycobacterium bovis BCG expressing the Sm14 antigen of Schistosoma mansoni protects mice from cercarial challenge. Infect Immun. 72: 3336-3343 ; and Varaldo, P.B., Leite, L.c.c., Dias, W.O., Miyaj, E.N., Armôa, G.R.G., Campos, A.S., Vilar, M.M., McFadden, J., Almeida, M.S., Tendler, M and McIntosh, D. (2006). Mycobacterial codon optimization of the gene encoding the Sm14 gene of Schistosoma mansoni in recombinant Mycobacterium bovis Bacille Calmette-Guérin enhances protein expression but not protection against cercarial challenge in mice. FEMS Medical Microbiology and Immunology, 48, 132-139.]

Several advantages are associated with the use of rBCG as an heterologous antigen presenting system [Ohara N and Yamada T (2001) Recombinant BCG vaccines. Vaccine. 19: 4089-4098 .]. In this context, Varaldo et al (2004) have previously reported that immunization with a single dose of a rBCG strain expressing Sm14 in fusion with the [beta]-lactamase of Mycobacterium fortuitum (rBCG/PL73-Sm14) conferred protection against challenge with S. mansoni cercaria equivalent to that obtained using three doses of rSm14 (Varaldo et al, 2004). The overall level of Sm14 expression obtained from this construction was relatively low in comparison to that recorded for other antigens using the same or similar expression vectors (Mederle I, Bourguin I, Ensergueix D, Badell E, Moniz-Pereira J, Gicquel B & Winter N (2002) Plasmidic versus insertional cloning of heterologous genes in Mycobacterium bovis BCG: impact on in vivo antigen persistence and immune responses. Infect Immun. 70: 303-314 .]. However, it should be noted that there is no consensus as to the minimum level of antigen production required for an rBCG strain to elicit protective immunity, but low level antigen expression has been considered to represent a potentially limiting factor (Kanekiyo M, Matsuo K, Hamatake M, Hamano T, Ohsu T, Matsumoto S, et al., (2005) Mycobacterial codon optimization enhances antigen expression and virus-specific immune responses in recombinant Mycobacterium bovis bacille Calmette-Guérin expressing human immunodeficiency virus type 1 Gag. J Virol. 79: 8716-8723 ; and, Kawahara M, Matsuo K, Nakasone T, Hiroi T, Kiyono H, Matsumoto S, et al (2002) Combined intrarectal/intradermal inoculation of recombinant Mycobacterium bovis bacillus Calmette-Guérin (BCG) induces enhanced immune responses against the inserted HIV-1 V3 antigen. Vaccine. 21: 158-166].

To address this problem there were developed two new rBCG constructs capable of express enhanced levels of the target antigen [Masters Thesis entitled Construca-o do M. bovis BCG recombinate Sm14r e avaliaca-o da sua capacidade contra esgistossomose no modelo murino. A.P.C. Argondizzo in 2005, in Oswaldo Cruz Foundation, Rio de Janeiro, Brasil). These constructs employed two plasmid vectors, the pUS977 and pAU5.

The pAU5 vector was developed in the course of the Masters Thesis entitled Avaliaçao da estabilidade estrutural e funcional de vetoes plasmidiais recombinantes bifuncionais (Escherichia coli - Mycobacterium) em Mycobacterium bovis BCG, J.P.S Santos in 2002, Oswaldo Cruz Foundation, Rio de Janeiro, oriented by Dr. Douglas McIntosh, visiting researcher of FIOCRUZ and Prof. Walter Martim R. Oelemann, teacher at Universidade Federal de Rio de Janeiro. The pAU5 vector is a variation on the plasmid pUS973 [Medeiros, M., Dellagostin, O.A., Armoa, G.R.G, Degrave, W.M., Mendonça-Lima, L., Lopes, M.Q., Costa, J.F., McFadden, J. G.M.B., and McIntosh, D. (2002) Comparative evaluation of Mycobacterium vaccae as a surrogate cloning host for use in the study of mycobacterial genetics. Microbiology 148, 1999-2009]. This plasmid (pAU5) demonstrated increased stability in comparison to pUS973 from which it is originated. The increased stability is due to a deletion of four base pairs immediately upstream of the promoter region in combination with the loss of the first base pair of the hsp60 promoter sequence. The thus-modified hsp60 sequence is termed hsp60*.

The ability of these rBCG constructs to confer protection to Swiss mice against cercarial challenge of S. mansoni was confirmed in two separate experiments [Master Thesis A.P.C. Argondizzo in 2005]. In this study, it was observed that the enhanced level of Sm14 expression (approximately 32 fold greater than that obtained with PL73-sm14 in the case of the rBCG construct transformed with the pAU5 vector), did not lead to an enhancement in the level of protection when compared to those recorded with the rBCG/PL73-Sm14 construct. Moreover, it is important to note that the rBCG/pAU5-Sm14 and rBCGr/pUS977-Sm14 strains also differed from the rBCG/PL73-Sm14 strain in terms of the sub-cellular location of the recombinant Sm14 protein. Specifically, the protein was produced within the bacteria cytoplasm in the case of rBCG-/pAU5-Sm14 rather than in the form of a membrane associated fusion protein as in the case of rBCG-/PL73-Sm14.

Taken as a whole, these data support the hypothesis that the recombinant Sm14 produced in the rBCGr cytoplasm, with expression driven by the modified hsp60 promoter present in the construction pAU5-Sm14, holds potential as a means of prophylaxis against schistosomiasis (rBCG/pAU5-Sm14).

All the plasmid constructs developed up to this point were based upon extrachromosomal, circular, plasmid DNA molecule containing a gene encoding resistance to the aminoglycoside antibiotic kanamycin. The kanamycin gene is present in these constructs to provide a means of positively selecting transformed bacteria (transformants) following the introduction of the plasmid into BCG. A secondary function of this gene is to ensure the maintenance of the plasmid during the growth of the bacterium in the liquid cultures, containing kanamycin, used to produce experimental vaccines.

However, the presence of the encoding gene of resistance to antibiotics does not provide selection for plasmid maintenance in vivo. In addition, the use of recombinant BCG carrying a plasmid containing a resistance encoding sequence to antibiotics for human vaccination raises safety concerns (Burlein JE, Stover CK, Offcut S & Hanson, M.S. (1994) Expression of foreign genes in mycobacteria. Washington DC: ASM Press .].

Clearly, the development of more stable plasmid vectors for heterologous antigen expression in BCG that do not carry antibiotic resistance markers would be desirable. In view of the predescribed it is an object of the present invention to provide an improved recombinant auxotrophic vaccinal strain, which is usable in the treatment of respective infections.

### Summary of the Invention

The inventive solution is obtained according to the independent claims. Additional advantageous features may be derived from dependent claims and following specification.

The present invention refers to a recombinant vaccinal strain of BCG Mycobacterium bovis expressing the Sm14 antigen of Schistosoma mansoni. The vaccinal strain of the present invention is employed to control infections caused by parasites, specially the Schistosoma mansoni. The vaccinal strain is an auxotrophic strain complemented to leucine derived from the genetic modification of BCG Pasteur sub-strain.

### Brief Description of the Figures

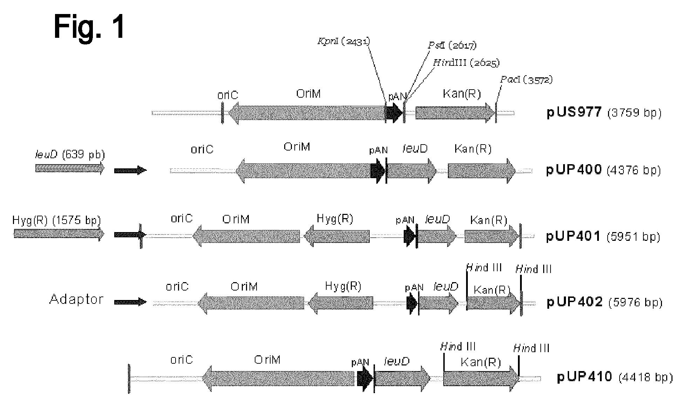

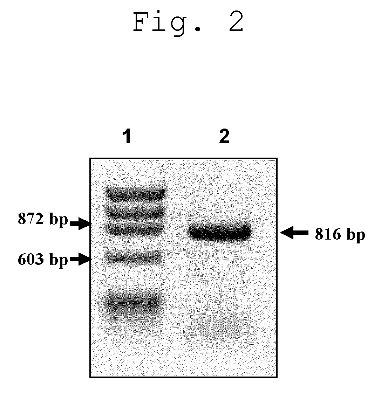

Figure 1 is a schematic representation of the development of complementary vector pUP410. Figure 2 shows the result of PCR to the amplification of the expression cassette hsp60*-Sm14 using the construct pAU5-Sm14 as DNA template.

### Detailed Description of the Invention

The present invention refers to the use of the BCG [Delta]leuD strain and the complementary vector pUP410 to obtain a vaccinal strain for the control of infections with Schistosoma mansoni and related parasites, based on expression of the Sm14 of S. mansoni in vivo.

A main feature of the present invention can be achieved through the construction of an expression system of the Sm14 antigen in BCG using auxotrophic complementation as selectable marker according to claim 1. The expression system used in the present invention was developed by the groups of Dr Johnjoe Mcfadden, School of Biological Sciences, University of Surrey, UK and Dr Odir Dellagostin, Biotechnology Centre, Federal University of Pelotas, Brasil, to the research project entitled Development of recombinant BCG multivaccine and complementary diagnostics for predominant parasitic and epizootic disease of ruminants in Latin America.

The components and features of this system of BCG employing auxotrophic complementation are a strain of BCG Pasteur which is auxotrophic with respect to the amino acid leucine (BCG [Delta]leuD) and a plasmid vector family encoding the product of the LeuD sequence which, as such is capable of complementing the auxotrophy of BCG to leucine. The auxotrophic BCG strain which is incapable of growth in media lacking leucine, was obtained by gene replacement of the LeuD sequence, present in the genome of BCG Pasteur, employing the methods reported by Parish, T. & and Stoker, N.G. (2000) [Use of a flexible cassette method to generate a double unmarked Mycobacterium tuberculosis tlyA plcABC mutant by gene replacement. Microbiology. 146: 1969-1975]. The complementation vectors are derived from vector pUS77 [Medeiros, M., Dellagostin, O.A., Armoa, G.R.G., Degrave, W.M, Mendonça-Lima, L., Lopes, M.Q., Costa, J.F., McFadden, J. G.M.B., and McIntosh, D. (2002) Comparative evaluation of Mycobacterium vaccae as a surrogate cloning host for use in the study of mycobacterial genetics. Microbiology 148, 1999-2009] and contains the LeuD gene which acts as complementary of the nutritional deficiency (auxotrophy) and as selectable marker, with the expression driven by the pAN promoter. In addition, these vectors possess unique restriction endonuclease sites which allow cloning of antigen expression cassettes into the vector and also possess a kanamycin resistance gene, necessary for the selection in E. coli, which can be further removed by restriction enzyme digestion followed by re-ligation prior to BCG transformation. The absence of leucine either in vitro growth medium (Middlebrooke growth medium 7H9 broth or 7H10 agar) or in vivo provides constant selective pressure which forces the rBCG to maintain the complementation plasmid. Details of the methods used to produce the BCG [Delta]leuD strain and a series of complementation vectors were described by Borsuk, S., et al. in Auxotrophic complementation as a selectable marker for stable expression of foreign antigens in Mycobacterium bovis BCG, Tuberculosis, Vol. 87, Magazine 6, pp 474-480, published online in September 24 2007 . The steps involved in the development of the complementation vector pUP410 which is derived from the complementation vectors family are represented in Figure 1.

According to what is represented in Figure 1, the steps to obtain the pUP410 vector are as follows: obtaining the leuD gene coding sequence by PCR using the primers leuDI (5'-AAT CTA GAA CAG CTA GGG GAT C-3' - SEQ ID NO:1), and leuDII (5'TCC TGCA GTT CTA CGC CTC A-3' - SEQ ID NO:2) and Mycobacterium bovis BCG P3 (Isogen) DNA as template; digestion of the amplified fragment (617 bp) with the enzymes Xbal and Pstl; and, subsequent cloning of pUS977 plasmid (Medeiros et al., 2002) originating the vector called pUP400.

The hyg R gene (1575 bp) was obtained from the PGOAL19 vector by digestion with the Kpnl enzyme [Parish, T & Stoker, N.G (2000). Use of a flexible cassette method to generate a double unmarked Mycobacterium tuberculosis tlyA plcABC mutant by gene replacement. Microbiology. 146: 1969-1975] and linked to the pU400 vector digested with the same enzyme. This proceeding resulted in the pU401 vector. To remove the kanamycin resistance gene by digestion with HindIII followed by re-ligation, an adaptor which carries the HindIII site and compatible end with Pacl was synthesized. One microgram of Adap F (5'GAT ATC AAG CTT AAG ACG CGT TAA3') and Adap R (5'TAA CGC GTC TTAAGC TTG ATA TCT A 3') sequences were hybridized by boiling for 1 min and then incubating for 3h at room temperature. Then, five hundred nanograms (ng) of the oligonucleotides were used in a linking reaction with 200 ng of the pUP401 vector digested with Pacl. The ligation product was heated at 70[deg.]C for 10 min, and immediately submitted to electrophoresis in 1% agarose gel and excised and eluted from the gel. One hybridization buffer (10 mM Tris-HC1 pH 8.5;100 mM NaCl; 1 mM EDTA) was added to the eluted DNA and heated at 80[deg.]C for 5 min. Then the DNA was allowed to cool to room temperature for 3 hours and five microliters were used for the transformation of the E. coli TOP10 strain. Recombinant clones were identified by digestion with Hindlll and by DNA sequencing. The resulting construct was named pUP402. The hyg<R> gene was then removed by digestion with Kpn1 resulting in the vector pUP410.

The invention will now be described by reference to the examples, which shall not be considered as limiting the present invention.

A. Construction of complemented auxotrophic rBCG Pasteur to leucine (BCGr Pasteur [Delta]leuD/p[Delta]Kan-hsp60*-Sm14)

The primers PSm14F (TAT ATA TAT ATA TAG GCG CCA CCA CAA CGA CGC GC - SEQ ID NO: 3) and PSm14R (CGC GGG CGC CTT AGG ATG ATC GTT TAT A - SEQ ID NO: 4) were designed to allow the amplification by PCR of the modified hsp60* promoter and Sm14 expression cassette using the vector pAU5-Sm14 as template. The resulting 816 bp amplicon obtained by PCR (Figure 2) was digested with the enzyme Nar1 and then cloned into the vector pUP410 digested with the same enzyme. The analysis of the DNA sequence confirmed that the expression cassette was inserted in the desired location. In Figure 2 the Lane 1 = Marker [phi]X174RF/HaeIII, and Lane 2 = hsp60*-Sm14 amplicon.

The ability of the construction pUP410-hsp60*-Sm14 to drive expression of the Sm14 antigen was assessed in the surrogate cloning host (Mycobacterium vaccae). Thus, five individual transformants selected on plates containing kanamycin were used to produce liquid cultures which were processed for expression analysis by immunoblotting as described by Medeiros et al, (2002). The antigen expression was detected using a mouse polyclonal anti-Sm14 antisera with antimouse IgG conjugated to alkaline phosphatase as secondary antibody. All five colonies were found to express Sm14 at levels equivalent to those seen in M. vaccae transformed with pAU5-Sm14 (expression positive control).

Then, the kanamycin resistance gene was removed from pUP410-hsp60*-Sm14 by digestion with HindIII restriction endonuclease, and the vector was re-circularized using T4 ligase giving rise to the construct p[Delta]K410-hsp60*-Sm14 which was used to transform the auxotrophic strain of BCG Pasteur (BCG [Delta]leuD). The transformants obtained were selected on plates of Middlebrook 7H10 agar (contains no leucine) and tested as to the ability of growth in the presence of kanamycin. Thereafter, the in vitro stability in terms of the ability to express the Sm14 protein of tree individual transformants sensible to kanamycin, was assessed over 60 generations in Middlebrook 7H9 broth (contains no leucine) and over 10 days within infected human and murine monocytes using previously reported methods (Varaldo et al, 2002).

To this purpose, murine or human monocytes were infected with two clones of stable BCG in vitro transformed with the construct p[Delta]K410-hsp60*-Sm14. The macrophages (5 x 105 per well) were previously cultivated on plates with 24 wells, using as culture means the RPMI 1640 supplemented with HEPES 10 mM; L-glutamine 2 mM; and 1% of bovine fetal sera. Afterwards the cell monolayers were exposed to levels equivalent to each BCG recombinant in order to obtain a multiplicity of infection (MOI: "multiplicity of infection") of approximately 10 bacterial cells per macrophage.

The mycobacterial inocula were prepared by culture dilution in logarithm phase up to the desired concentration, based on the direct counting by optical microscopy. Samples (six wells per culture) were collected 4 h, 24 h, 5 days and 10 days after the macrophages exposure to BCG recombinant. The collection after lysis of the monolayers with 0.1 % of Tween in distilled water (200 [micro]L per well) was followed by serial dilutions of the lysed, and subsequent inoculation of agar 7H10 plates or in the same means supplemented with kanamycin.

The levels of mycobacterial growth, observed from the analysis of the samples collected after 4 hours of exposal, were used to calculate the relative infectious ability. The values obtained from the counting of units which form colonies of other points were used to calculate the level of intracellular persistence. The functional stability (expression of the antigen Sm14) was analyzed by immunoblotting according to the protocol previously described (Medeiros 2002).

In all the cases, it has been noted that the transformants express the protein Sm14 in levels equivalent to those seen in M. bovis BCG transformed with pAU5-Sm14 (expression positive control). The expression system stability, therefore, was confirmed both in the non-cellular means and in culture of murine and human cells.

B. BCGr Pasteur [Delta]leuD/p[Delta]Kan-hsp60*-Sm14 protects mice against S. Mansoni cercarial challenge.

The rBCG Pasteur [Delta]leuD/p[Delta]Kan-hsp60*-Sm14 ability to protect mice against live S. Mansoni cercaria was assessed in two separate experiments. Each experiment employed 6 experimental groups (Table 1), each group containing 10 females of Swiss mice with 4 weeks of age. The vaccination efficacy with only one dose administered intranasally (1 x 10<6> cfu = standard dose; or 1 x 10<2> cfu = low dose) of rBCG-(p[Delta]K21-Sm14) was compared to that achieved by the administration of similar single doses of rBCG-(pAU5-Sm14) or 3 doses of rSm14 protein (10 [micro]g in alumina hydroxide) at the paw as previously described in Varaldo et al., (2004).

The final recombinant protein dose was administered on the same day as the vaccine single doses based on BCGr. For protection assays, the mice were subcutaneously (sc) challenged with 100 S. mansoni cercaria im 30 days (experiment 1) or in 60 days (experiment 2) after the initial immunizations and perfused 45 days after the challenge. The parasitic burden and protection levels in different challenge groups were calculated according to the previously described in Tendler et al., 1996. The statistic importance was assessed through the T test of Student.

**Table 1 - Immunization protocol employed to evaluate the capacity of rBCG Pasteur [Delta]leuD/p[Delta]K410-hsp60*-Sm14 to confer protection against challenge with S. Mansoni cercaria**

| Experimental Group | Immunization (day 0) | Colony-Forming-Unit of BCG |
|---|---|---|
| A | Saltine | ------- |
| B | rBCG-[Delta]LeuD/p[Delta]K410-hsp60*-Sm14)^{a} | 1 x 10⁶ |
| C | rBCG [Delta]LeuD/p[Delta]K410-hsp60*-Sm14)^{b} | 1 x 10² |
| D | rBCG/pAUS-Sm14^{a} | 1 x 10⁶ |
| E | rBCG/pAUS-Sm14^{b} | 1 x 10² |
| F | Sm14r (10 [micro]g) in alumen^{c} (3 individual and consecutive doses one week apart) | ------- |

| | | |
|---|---|---|
| a = standard dose b = low dose c = alumina hydroxide, as previously reported (Varaldo et al., 2004). | | |

The challenge results are represented on Table 2. It can be noted that all the vaccinated groups showed a significant decrease in the parasitic burden in relation to the control animals (saline group). This result was clear on both after day 30 and day 60 following the vaccination. In general, this data show clearly that rBCG Pasteur [Delta]leuD/p[Delta]Kan-hsp60*-Sm14 is capable of inducing a protective immune response in 30 days and that this response remains effective 60 days after the immunization. Besides, the new vaccinal strain was as protective as the BCGr/pAU5-Sm14 strain according to the above described on the previous art. At last, the protection level achieved with one single dose administered intranasally (both 1 x 10<6> cfu = standard dose and 1 x 10<2> cfu = low dose) of BCGr Pasteur [Delta]leuD/p[Delta]Kan-hsp60*-Sm14 was capable of providing a protection level equivalent to that obtained as 3 doses of recombinant Sm14 protein formulated with alumina hydroxide as adjuvant.

**Table 2 - Evaluation of protection as measured in terms of the reduction in worm burden in comparison to PBS (saline) controls in immunized Swiss mice at 30 and days post vaccination**

| Immunized with | Reduction in worm burden % (30 days) | Reduction in worm burden % (60 days) |
|---|---|---|
| Saline | 0 | 0 |
| rBCG-(p[Delta]K410-hsp60*-Sm14)^{a} | 46.0 | 63.0 |
| rBCG([p[Delta]K410-hsp60*-Sm14)^{b} | 57.4 | 57.4 |
| rBCG (pAU5-Sm14)^{a} | 43.8 | 61.2 |
| rBCG(pAU5-Sm14)^{b} | 39.6 | 66.4 |
| rSm14(10 [micro]g) in alum^{c} (3 individual and consecutive doses one week apart) | 54.9 | 57.6 |

| | | |
|---|---|---|
| a = standard dose b = low dose c = as previously reported (Varaldo et al., 2004). | | |

Following what was reported and exemplified above, the applicant confirm the efficacy of the BCG Pasteur [Delta]leuD strain and the complementary vector p[Delta]K410-hsp60*-Sm14 to obtain the vaccinal strain for the control of infections with Schistosoma mansoni and related parasites based on the native Sm14 antigen expression.

The specific embodiments herein described and the explained advantageous additional aspects should not be considered as limitation. It should be noted that the invention is not limited to those specific embodiments, and those skilled in the art should note that any additinal, particular characteristic here introduced, must be understood only as a description to render the invention an easier comprehension. Within the scope of independent claims additional features or measures are possible without leaving the claimed subject-matter.

## Claims

1. A recombinant auxotrophic vaccinal BCG Pasteur strain complemented to leucine amino acid expressing a Schistossoma mansoni Sm14 protein, i. e. rBCG Pasteur [Delta]leuD/p[Delta]K410-hsp60*-Sm14, said vaccinal strain being obtained according to the following steps:
(a) amplification by PCR of a fragment containing an Sm14 expression cassette containing a modified hsp60* promoter, using
- a pAU5-Sm14 vector as template, said vector having a modified hsp60 sequence which has a deletion of four base pairs immediately upstream of the promoter region in combination with the loss of the first base pair of the hsp60 promoter sequence and is designated therefor as hsp60*, and
- primers PSm14F, i.e. TAT ATA TAT ATA TAG GCG CCA CCA CAA CGA CGC GC
- SEQ ID NO:3 and PSm14R, i. e. CGC GGG CGC CTT AGG ATG ATC GTT TAT A - SEQ ID NO:4;
(b) digestion with a NAR1 enzyme of an amplicon of 816 bp, i. e. hsp60*-Sm14, obtained by the PCR amplification of step (a), providing a hsp60*-Sm14 amplicon;
(c) cloning of the hsp60*-Sm14 amplicon obtained in step (b) in a pUP410 vector digested with the Nar1 enzyme, giving rise to a pUP410-hsp60*-Sm14 construct;
(d) evaluating an ability of the construct pUP410-hsp60*-Sm14 to express the Sm14 protein in Mycobacterium vaccae;
(e) removing the kanamycin resistance gene from the pUP410-hsp60*-Sm14 construct by digestion with an endonuclease restriction HindIII; and
(f) re-circularization of the digested pUP410-hsp60*-Sm14 construct without the kanamycin resistance gene using T4 ligase to produce the digested pUP410-hsp60*-Sm14 construct, which is used to transform a BCG Pasteur auxotrophic strain, i. e. BCG [Delta]leuD, to produce a recombinant auxotrophic BCG Pasteur strain complemented to leucine amino acid expressing a Schistossoma mansoni Sm14 protein, i. e. BCG Pasteur [Delta]leuD/p[Delta]K410-hsp60*-Sm14.

2. BCG Pasteur [Delta]leuD/p[Delta]K410-hsp60*-Sm14 strain of claim 1 for use in the treatment of schistosomiasis.

3. BCG Pasteur [Delta]leuD/p[Delta]K410-hsp60*-Sm14 strain of claim 1 for use in the treatment of fascioliasis.

4. BCG Pasteur [Delta]leuD/p[Delta]K410-hsp60*-Sm14 strain of claim 1 for use in the treatment of tuberculosis.

5. BCG Pasteur [Delta]leuD/p[Delta]K410-hsp60*-Sm14 strain of claim 1 for use in the treatment of neoplasias.

## Patentansprüche

1. Rekombinanter auxotropher vakzinaler BCG Pasteur Stamm ergänzend zur Aminosäure Leucin, ein Schistosoma mansoni Sm14-Protein exprimierend, nämlich rBCG Pasteur [Delta]leuD/p[Delta]K410-hsp60*-Sm14, wobei dieser vakzinale Stamm erhalten wird durch die nachfolgenden Schritte:
(a) Amplifikation durch PCR eines Fragments enthaltend eine Sm14 Exprimier-Kassette, enthaltend einen modifizierten hsp60*Promoter, verwendend
- einen pAU5-Sm14 Vektor als Templat, wobei dieser Vektor eine modifizierte hsp60-Sequenz hat, die eine Deletion von vier Basenpaaren unmittelbar oberhalb einer Region des Promoters hat in Kombination mit dem Verlust des ersten Basenpaares der hsp-Promoter-Sequenz, und die daher mit hsp60* bezeichnet wird, und
- Primer PSm14F, nämlich TAT ATA TAT ATA TAG GCG CCA CCA CAA CGA CGC GC
- Sequenz ID NO:3 und PSm14R, nämlich CGC GGG CGC CTT AGG ATG ATC GTT TAT A-SEQ ID NO:4;
(b) Digestion mit einem NAR1-Enzym eines Amplikons von 816 bp, nämlich hsp60*-Sm14, erhalten durch PCR-Amplifikation gemäß Schritt (a), zur Verfügung stellen eines hsp60*-Sm14-Amplikons;
(c) Klonen des in Schritt (b) erhaltenen hsp60*-Sm14-Amplikons in einem pUP410 Vektor, digestiert mit dem Nar1-Enzym, wobei ein pUP410-hsp60*-Sm14-Konstrukt gebildet wird;
(d) evaluieren einer Fähigkeit des Konstrukts pUP410-hsp60*-Sm14, das Sm14-Protein in Mycobakterium vaccae zu exprimieren;
(e) Entfernen des Kanamycin-Resistenz-Gens aus dem pUP410-hsp60*-Sm14-Konstrukt durch Digestion mit einer Endonuklease-Restriktions HindIII; und
(f) Rezirkulation des digestierten pUP410-hsp60*-Sm14-Konstrukts ohne das Kanamycin-Resistenz-Gen unter Verwendung von T4-Ligase zur Herstellung des digestierten pUP410-hsp60*-Sm14-Konstrukts, welches verwendet wird zum Transformieren eines auxotrophen BCG-Pasteur-Stamms, nämlich BCG Pasteur [Delta]leuD, zur Herstellung eines rekombinanten auxotrophen BCG Pasteur-Stamms, ergänzt durch die Aminosäure Leucin, welcher ein Schistosoma mansoni Sm14-Protein exprimiert, nämlich BCG Pasteur [Delta]leuD/p[Delta]K410-hsp60*-Sm14.

2. BCG Pasteur [Delta]leuD/p[Delta]K410-hsp60*-Sm14-Stamm nach Anspruch 1 für die Verwendung zur Behandlung von Schistosomiasis.

3. BCG Pasteur[Delta]leuD/p[Delta]K410-hsp60*-Sm14-Stamm nach Anspruch 1 für die Verwendung zur Behandlung von Fasziolose.

4. BCG Pasteur [Delta]leuD/p[Delta]K410-hsp60*-Sm14-Stamm nach Anspruch 1 für die Verwendung zur Behandlung von Tuberkulose.

5. BCG Pasteur [Delta]leuD/p[Delta]K410-hsp60*-Sm14-Stamm nach Anspruch 1 für die Verwendung zur Behandlung von Neoplasien.

## Revendications

1. Souche de BCG Pasteur auxotrophe recombinant complémenté pour l'acide aminé leucine exprimant une protéine Sm14 de Schistossoma mansoni, plus précisément rBCG Pasteur [Delta]leuD/p[Delta]K410-hsp60*-Sm14, ladite souche vaccinale étant obtenue selon les étapes suivantes :
(a) amplification par PCR d'un fragment contenant une cassette d'expression de Sm14 contenant un promoteur hsp60* modifié, en utilisant
- un vecteur pAU5-Sm14 en tant que matrice, ledit vecteur ayant une séquence de hsp60 modifiée qui porte une délétion de quatre paires de bases immédiatement en amont de la région du promoteur en combinaison avec la perte de la première paire de bases de la séquence du promoteur hsp60 et est par conséquent désignée hsp60*, et
- les amorces PSm14F, plus précisément TAT ATA TAT ATA TAG GCG CCA CCA CAA CGA CGC GC - SEQ ID NO : 3 et PSm14R, plus précisément CGC GGG CGC CTT AGG ATG ATC GTT TAT A - SEQ ID NO: 4 ;
(b) digestion avec une enzyme NAR1 d'un produit d'amplification de 816 pb, plus précisément hsp60*-Sm14, obtenu par amplification par PCR de l'étape (a), permettant d'obtenir un produit d'amplification hsp60*-Sm14 ;
(c) clonage du produit d'amplification hsp60*-Sm14 obtenu dans l'étape (b) dans un vecteur pUP410 digéré avec l'enzyme Nar1 permettant d'obtenir une construction pUP410-hsp60*-Sm14 ;
(d) évaluation d'une capacité de la construction pUP410-hsp60*-Sm14 à exprimer la protéine Sm14 dans Mycobacterium vaccae ;
(e) retrait du gène de résistance à la kanamycine de la construction pUP410-hsp60*-Sm14 par digestion avec une endonucléase de restriction HindIII ; et
(f) re-circularisation de la construction pUP410-hsp60*-Sm14 digérée sans le gène de résistance à la kanamycine en utilisant une ligase T4 pour produire la construction pUP410-hsp60*-Sm14 digérée, qui est utilisée pour transformer une souche auxotrophe de BCG Pasteur, plus précisément BCG [Delta]leuD, pour produire une souche de BCG Pasteur auxotrophe recombinant complémenté pour l'acide aminé leucine exprimant une protéine Sm14 de Schistossoma mansoni, plus précisément BCG Pasteur [Delta]leuD/p[Delta]K410-hsp60*-Sm14.

2. Souche de BCG Pasteur [Delta]leuD/p[Delta]K410-hsp60*-Sm14 selon la revendication 1, pour son utilisation dans le traitement de la schistosomiase.

3. Souche de BCG Pasteur [Delta]leuD/p[Delta]K410-hsp60*-Sm14 selon la revendication 1, pour son utilisation dans le traitement de la fasciolose.

4. Souche de BCG Pasteur [Delta]leuD/p[Delta]K410-hsp60*-Sm14 selon la revendication 1, pour son utilisation dans le traitement de la tuberculose.

5. Souche BCG Pasteur [Delta]leuD/p[Delta]K410-hsp60*-Sm14 selon la revendication 1, pour son utilisation dans le traitement des néoplasies.
